# EUROPÄISCHE PATENTSCHRIFT

(11) **EP 0 906 096 B1**
(45) Veröffentlichungstag und Bekanntmachung des Hinweises auf die Patenterteilung: **26.06.2002**
(21) Anmeldenummer: 97920703.2
(22) Anmeldetag: 16.04.1997
(51) Int. Cl.: A61K 31/40, A61P 29/00

(54) **VERWENDUNG VON HYDROXYPROLINEN ZUR HERSTELLUNG EINES ARZNEIMITTELS ZUR HEMMUNG DER LEUKOZYTENINVASION IN GEWEBE**
USE OF HYDROXYPROLINES FOR THE MANUFACTURE OF A MEDICAMENT FOR INHIBITING LEUKOCYTE INVASION OF TISSUES
UTTILISATION DE HYDROXYPROLIENS POUR LA FABRICATION D'UN MEDICAMENT POUR L'INHIBITION DE L'INVASION DES TISSUS PAR DES LEUCOCYTES

(30) Priorität: 16.04.1996 DE 19614919; 25.04.1996 DE 19616581
(43) Veröffentlichungstag der Anmeldung: 07.04.1999
(73) Patentinhaber: Chephasaar Chem.-Pharm. Fabrik GmbH, 66386 St. Ingbert (DE)
(72) Erfinder: BRUNE, Kay, D-91080 Marloffstein/Rathsberg (DE); HALLMANN, Rupert, D-91341 Röttenbach (DE)
(74) Vertreter: Böhm, Brigitte, Dipl.-Chem. Dr.
(86) Internationale Anmeldenummer: EP9701909
(87) Internationale Veröffentlichungsnummer: WO9738690

(56) Entgegenhaltungen:
- WO-A-91/18896
- DE-A- 2 139 476
- DE-B- 1 795 327
- GB-A- 2 171 302
- US-A- 3 932 638
- US-A- 5 364 775
- DATABASE WPIL, AN 74-16 169v, DERWENT PUBLICATIONS LTD., London; & JP,A,48 085 718 (AJINOMOTO CO INC),
- HELWIG/OTTO ARZNEIMITTEL, "Antirheumatika", 8. Auflage, Band 1, Juli 1992, Seiten 14/44-14/45 in Ver- bindung mit Seite 14/1, Handelspr parat AMP 200 (R).

## Beschreibung

Gegenstand der vorliegenden Erfindung ist die Verwendung von Hydroxyprolinderivaten der Formel (I) zur Herstellung eines Arzneimittels zur Hemmung der Einwanderung von Leukozyten in krankhaft veränderte Gewebe. Dieses neuartige Verfahren unter Verwendung des erfindungsgemäßen Mittels kann dazu dienen, akute und chronische Gewebsveränderungen, die durch die Einwanderung von Granulozyten bedingt sind, zu verhindern oder zu vermindern und dadurch Krankheiten zu verhindern oder zu heilen.

Die Auswanderung von Blutleukozyten aus dem Blutstrom in ein Gewebe ist ein zentraler Vorgang beim Ablauf jeder Entzündungsreaktion (T. Springer (1994), Cell 76, 301 - 314, Traffic signals, und T. Tedder (1995), FASEB J. 9, 866 - 873, The Selectins). Auswandernde Granulozyten sind insbesondere an der Ausbildung akuter Entzündungsreaktionen (M.K. Robinson, PE Stephens (1992), Curr. Opin. Biotechnicol. 3, 662 - 667; J. Panes, DN Granger (1994), Dig. Dis. 12, 232-241; und E. Christophers, HJ Rowert(1994), Hautarzt 45, 658-669), aber auch z. B. Reperfusionsschäden (P.R. Hansen (1995). Circulation 91, 1872-1885) beteiligt. Die Blutgefäßwand und vornehmlich das Endothel als luminale Grenzschicht des Gefäßes regulieren neben Oberflächenstrukturen der Leukozyten maßgeblich die Extravasation der Leukozyten, die als Kaskade von Ereignissen abläuft (T. Springer (1994), Cell 76, 301 - 314, Traffic signals, und T. Tedder (1995), FASEB J. 9, 866 - 873, The Selectins):
1. Die Leukozyten lagern sich an das Endothel an,
2. wandern durch die Endothelschicht hindurch,
3. passieren die Basalmembran des Endothels, und können erst dann
4. das Gewebe lokal infiltrieren.

Die Kenntnis der Adhäsionsmoleküle, die auf der Seite der Endothelzelle sowie auch auf den Leukozyten an dieser Kaskade der Adhäsion und Migration beteiligt sind, ist in den letzten Jahren aufgrund der vermehrten Forschung gewachsen. Die Analyse der Adhäsionsmechanismen ist durch die in-vitro-Kultur von Endothelzellen stark begünstigt worden. Diese Kulturen erlauben die funktionelle Analyse der Interaktion zwischen dem Endothel und Leukozyten des Blutstroms unter Ausschluß von dritten Zelltypen und haben zur Charakterisierung aller bisher bekannten Adhäsionsmoleküle geführt. Drei Gruppen von Adhäsionsmolekülen sind an dem Vorgang maßgeblich beteiligt: Selectine, Integrine und Mitglieder der Immunglobulin-Superfamilie (T. Springer (1994), Cell 76, 301 - 314, Traffic signals, und T. Tedder (1995), FASEB J. 9, 866 - 873, The Selectins).

Leukozyten des Blutstroms binden im gesunden Organismus nicht an das Endothel der Gefäßwand und verlassen das Gefäßbett gar nicht oder nur in geringem Umfang. Vor allem im Gefolge einer Zirkulationsstörung, eines traumatischen Ereignisses oder einer andersartigen Gewebeschädigung adhärieren Leukozyten an dieses Endothel und verlassen das Gefäßbett.

Eine Vielzahl von Krankheiten ist charakterisiert durch die akute Einwanderung von granulozytären Leukozyten aus dem Gefäßsystem ins Gewebe (M.K. Robinson, PE Stephens (1992), Curr. Opin. Biotechnicol. 3, 662 - 667; P.R. Hansen (1995), Circulation 91, 1872 - 1885; J. Panes, DN Granger (1994), Dig. Dis. 12, 232 - 241 und E. Christophers, HJ Rowert (1994), Hautarzt 45, 658 - 669). Es handelt sich dabei zum einen um akut verlaufende, entweder zum Tod oder zur alsbaldigen Heilung führende Erkrankungen, zum anderen aber auch um akut einsetzende, aber chronische Verläufe aufweisende Krankheitszustände. Die pathophysiologisch bedeutende Rolle der granulozytären Einwanderung ist insbesondere definiert worden für die folgenden akuten oder akut beginnenden, aber chronisch verlaufenden Krankheitszustände:
**Akut verlaufende Krankheitszustände:**
   a) Abstoßung von Gewebetransplantaten;
   b) postischämische Reperfusionsschäden wie z. B. beim Herzinfarkt;
   c) akute Entzündungszustände aller Gewebe,
**Akut einsetzende und chronisch verlaufende granulozytär geprägte Krankheitszustände:**
   a) Neurodermitis und Psoriasis,
   b) entzündliche Darmerkrankungen (Ileitis regionalis, Colitis ulcerosa),
   c) posttraumatische und postoperative Entzündungen,
   d) akute Schübe der rheumatoiden Arthritis.

Es ist dabei davon auszugehen, dass für alle diese Krankheitsbilder der Auslöser entweder eine Mikrozirkulationsstörung oder eine durch eine externe Noxe, z.B. Trauma, oder lokalisierte Abwehrreaktion, z.B. Infektion, ausgelöste Gewebeschädigung mit veränderter Durchblutung und Leukozytenadhäsion, -invasion und -aktivierung ist. Es entstehen Gewebeschäden, die sich letztendlich in einer Vielzahl von Erkrankungen oder Folgeschäden manifestieren, jedoch alle auf einen grundlegenden Mechanismus zurückgehen. Die Prophylaxe bzw. Behandlung zur Heilung oder Verhinderung von weitergehenden Schäden kann daher für sehr unterschiedliche Erscheinungsbilder von Schäden mit dem gleichen Mittel erfolgen.

Aufgabe der vorliegenden Erfindung war es daher, ein Arzneimittel bereitzustellen, welches die Invasion von Leukozyten in Gewebe zumindest stark verringert und damit die genannten oder auf derselben Ursache beruhende Krankheiten bzw. Krankheiten, bei denen die Invasion von Leukozyten in Gewebe eine negative Begleiterscheinung ist, zu verhindern oder zu heilen oder zumindest günstig zu beeinflussen.

Gelöst wird diese Aufgabe durch die Verwendung von Hydroxyprolinderivaten der Formel (I) zur Herstellung eines pharmazeutischen Mittels zur Hemmung der Invasion von Leukozyten in Gewebe, welches dadurch gekennzeichnet ist, daß es ein Hydroxyprolinderivat der Formel (I), 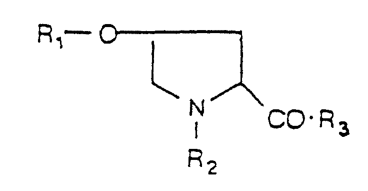 wobei R₁ und R₂ gleich oder verschieden sein können und Wasserstoff, Alkylgruppen mit 1 bis 10 C-Atomen oder eine Acylgruppe mit 1 bis 8 C-Atomen und R₃ eine Hydroxy-, Alkoxy- oder Aminogruppe, welche 1 oder 2 Alkylsubstituenten mit 1 bis 4 C-Atomen als auch 1 oder 2 Arylsubstituenten mit 6 bis 10 C-Atomen haben kann, bedeuten oder ein pharmazeutisch verträgliches Salz davon enthält.

Bei der Analyse der Interaktionen zwischen Endothelzellen und Leukozyten wurden auch die pharmakologischen Einflüsse einer Reihe von Zuckern und Aminosäurederivaten untersucht. Dabei wurde im Rahmen der vorliegenden Erfindung festgestellt, dass Hydroxyprolin-Derivate, die der obigen generellen Formel (I) entsprechen, die Adhäsion der Granulozyten an die Endothelzellen hemmen.

Als besonders wirksam zeigten sich die an R₂ acetylierten Verbindungen, die eine signifikante, noch bei 10⁻⁹ molarer Konzentration meßbare, Hemmung der Granulozytenadhäsion an Endothelien aufweisen. Die Substitution an R₁ mit z.B. COCH₃ reduzierte die Löslichkeit der Verbindungen, vermittelte aber bei niedriger Konzentration einen ähnlichen, wenn auch geringen Effekt wie die Substitution mit H (siehe Beispiel 1, Tab. 1).

In einer bevorzugten Ausführungsform der Erfindung enthält daher das erfindungsgemäße Mittel ein Hydroxyprolinderivat mit R₁ und R₂ gleich H, Alkyl- oder Acylgruppe mit 1 bis 3 C-Atomen und R₃ = OH, und ganz besonders bevorzugt N-Acetylhydroxyprolin oder pharmazeutisch verträgliche Salze davon.

Das erfindungsgemäße Verwendung bewirkt, daß insbesondere die Funktion der "Adhäsionsmoleküle" der Granulozyten gehemmt wird. Auch andere Leukozyten, wie Lymphozyten, und Monozyten zeigten eine verringerte Adhäsion an Endothelien. Auch bei Entzündungen bewirkte die Applikation des erfindungsgemäßen Mittels eine deutlich reduzierte Invasivität der Granulozyten. Ähnlich konnte bei allergischen Entzündungen in der späten Phase eine Verringerung der Invasivität der Granulozyten beobachtet werden.

Zusammenfassend läßt sich feststellen, daß die Verwendung der erfindungsgemäßen Hydroxyprolinderivate deutliche Hemmwirkungen auf die Adhäsion und Immigration von neutrophilen Granulozyten haben. Dieser Effekt betrifft andere Leukozyten ebenso, wenn auch in geringerem Umfang. Der Effekt beruht vermutlich auf der Hemmung der Funktion der granulozytären Adhäsionsmoleküle, er tritt nur akut auf, ist spezifisch für Leukozyten, insbesondere Granulozyten, und läuft auch beim vollkommenen Fehlen von interzellulärer Matrix, d.h. von Bindegewebsgrundstrukturen, ab.

Diese Wirkung ist daher verschieden von der schon früher festgestellten Wirkung von Hydroxyprolinderivaten auf entzündetes Bindegewebe (DE 1 795 327 B2, DE 2 139 476 A1, GB 1 246 141 C, US 3 932 638), welche über eine Beschleunigung der Proteinsynthese im Bindegewebe, d.h. eine verstärkte Kollagenbildung, verläuft (Kalbhen et al., Z. Rheumatologie 46, 136 -142 (1987)). Ein Vertreter dieser Gruppe, das N-Acetyl-L-hydroxyprolin (INN Oxaceprol), wird daher bei degenerativen Gelenkserkrankungen, Arthrosen, Arthritis und entzündlichen Bindegewebserkrankungen eingesetzt. Es ist dieser Literatur nicht zu entnehmen, daß auch bei anderen entzündlichen Erkrankungen, bei denen die Kollagenbildung keine Rolle spielt, eine positive Wirkung der Hydroxyprolinderivate erwartet werden kann.

Die Möglichkeit, durch die Applikation von im erfindungsgemäßen Mittel enthaltenen Hydroxyprolinderivaten prophylaktisch das Auftreten von Krankheitszuständen zu verhindern, oder aber bei chronischen Verläufen durch therapeutische Applikation von insbesondere N-acetylierten Hydroxyprolinderivaten den Verlauf von Krankheitszuständen positiv zu beeinflussen, welche durch Gewebeschäden auf der Basis der Invasion und Aktivierung von Leukozyten entstehen, stellt ein neuartiges Therapieprinzip dar.

Ein weiterer Gegenstand der vorliegenden Erfindung ist daher die Verwendung eines erfindungsgemäßen Mittels zur Prophylaxe und Heilung von Gewebeschäden, welche durch Invasion und Aktivierung von Leukozyten verursacht sind. In bevorzugten Ausführungsformen findet das erfindungsgemäße Mittel Verwendung zur Prophylaxe oder Behandlung von
a) akuten Abstoßungsreaktionen von Transplantaten,
b) Gewebeschäden infolge von Herzinfarkt, Thrombosen, Ebolien,
c) der Verhinderung und Linderung von akuten Schüben exerzerbierender Hautkrankheiten wie Neurodermitis, Psoriasis,
d) posttraumatische Entzündungen,
e) postoperative Entzündungen,
f) akute Schübe chronischer intestinaler Entzündungen, wie z.B. Colitis ulcerosa und chronischer Krankheit, und
g) septischen Erscheinungsformen.

Sowohl bei den unter a) aufgeführten Abstoßungsreaktionen von Transplantaten als auch bei den unter e) genannten postoperativen Entzündungen dürfte die Entstehungsursache in der operationsbedingten lokalen Blutverarmung und der damit einhergehenden Minderdurchblutung zu sehen sein. Prophylaktisch kann daher in solchen Fällen das erfindungsgemäße Mittel vor oder nach der Operation verabreicht werden, um Schäden zu vermeiden oder zu minimieren.

In gewisser Weise ist die Situation bei posttraumatischen Entzündungen ähnlich, da während des traumatischen Ereignisses ebenfalls die Mikrozirkulation gestört wird.

Eine weitere Verwendung des erfindungsgemäßen Mittels liegt in der Behandlung des septischen Schocks. Es hat sich gezeigt, dass im Verlauf einer Sepsis Leukozyten massiv in Organen und insbesondere in der Lunge an das Endothel der Blutgefäße adherieren. Auch hierbei kommt es zu Gewebeschäden, die dann ein Versagen von Organen zur Folge haben können. Daher ist eine prophylaktische oder heilende Anwendung des erfindungsgemäßen Mittels geboten.

Die Verwendung des erfindungsgemäßen Mittels und insbesondere von N-Acetylhydroxyprolin erfolgt vorzugsweise in Dosierungen von 200 bis 5000 mg. Diese Dosierung kann durch einmalige oder mehrmalige Verabreichung erfolgen. Besonders bevorzugt sind Dosierungen von 400 bis 1200 mg/Tag, entsprechend ca. 5 bis 20 mg/kg Körpergewicht pro Tag. Die Verabreichung kann in jeder geeigneten Form, wie enteral und parenteral, systemisch und topisch in allen Darreichungsformen erfolgen.

Die folgenden Beispiele sollen in Verbindung mit den Abbildungen die Erfindung weiter erläutern:
Abb. 1 zeigt hierbei den Einfluß der molaren Konzentration von N-Acetylhydroxyprolin auf die Adhäsion von Leukozyten an Endothelien nach Aktivierung durch LPS.
Abb. 2 zeigt den Zeitverlauf der Wirkung von N-Acetylhydroxyprolin auf die Granulozytenadhäsion an Endothelzellen.
Abb. 3 zeigt die Leukozytenadhärenz in postkapillaren Venolen.

### Beispiel 1

Gemäß M. Hahne et al. (1993), J. Cell. Biol. 121, 655-664 wurde die IC₅₀-Konzentration von Hydroxyprolinderivaten, die zu einer 50 %igen Reduktion der Adhäsion von Granulozyten an einer Endothelschicht führte, bestimmt. Als besonders wirksam zeigte sich die an R₂ acetylierte Verbindung.

**Tabelle 1:**

| Struktur und Wirkung von Hydroxyprolinderivaten | | | |
|---|---|---|---|
| R₁ | R₂ | R₃ | IC₅₀ |
| H | COCH₃ | OH | ∼ 10⁻⁷ (noch meßbar bei 10⁻⁹) |
| H | H | OH | > 10⁻⁵ |
| H | H | NH₂ | > 10⁻⁵ |
| COCH₃ | COCH₃ | OH | ∼ 10⁻⁶ |

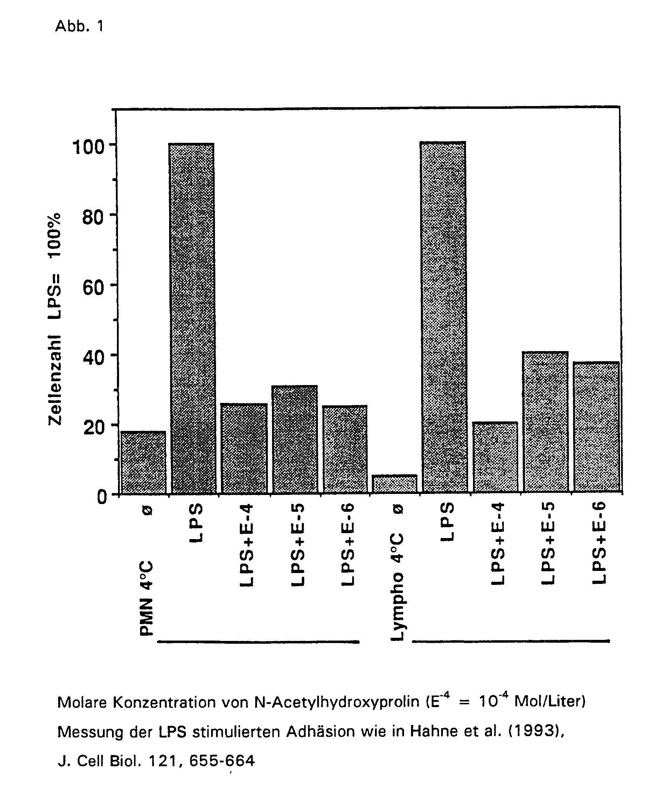

Eine weitere Analyse des Befundes zeigte, daß es insbesondere die Funktion der "Adhäsionsmoleküle" der Granulozyten war, die durch die genannten Substanzen gehemmt wurden. Auch andere Leukozyten (Lymphozyten, Monozyten) zeigten eine verminderte Adhäsion an Endothelien nach Aktivierung durch LPS. Diese Wirkung war allerdings nicht konstant nachweisbar. Der granulozytäre Effekt war deutlich anhaltender und konsistenter (Abb. 1).

### Beispiel 2:

Dieser überraschende Befund ließ sich durch eine Reihe von ex-vivo- Versuchen am Menschen bestätigen: Nach der ein- oder mehrmaligen Einnahme von 400 - 800 mg N-Acetylhydroxyprolin zeigten die Granulozyten von Probanden eine reduzierte Adhäsion an Endothelien. Dieser Effekt hielt über 24 - 48 Stunden an, geringgradige Effekte auf Lymphozyten und Monozytenadhäsion verschwanden hingegen nach wenigen Stunden. Die Funktion der Blutplättchen war nicht tangiert.

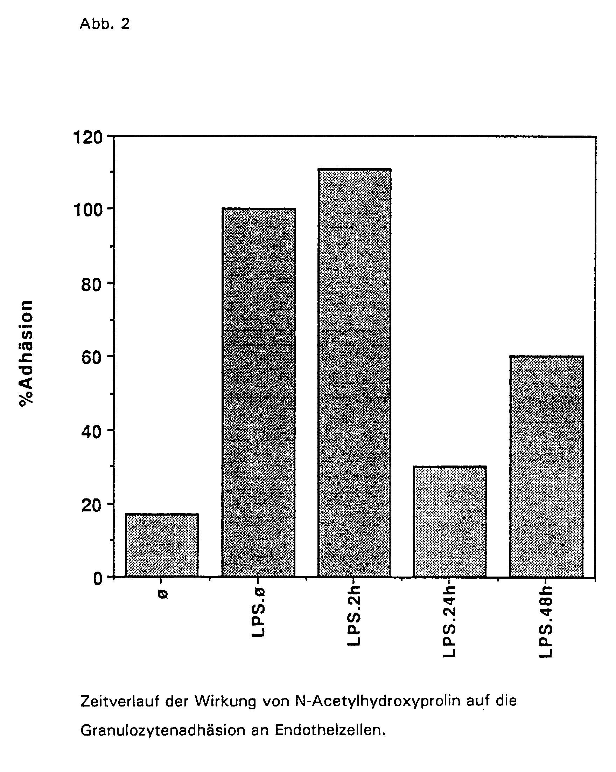

Abb. 2 zeigt das Ergebnis der folgenden Untersuchung:

Gesunde Probanden erhielten eine einmalige Dosis von N-Acetylhydroxyprolin zum Zeitpunkt 0. Unmittelbar vorher und zu den genannten Zeitpunkten wurde Blut abgenommen und die Leukozyten mit Hilfe der Lymphoprep-Separation (Histoprep, Biologische Arbeitsgemeinschaft GmbH, Lich, Germany) präpariert. Die Adhäsion wurde wie in M. Hahne et al. (1993), J . Cell Biol. 121, 655 - 664 beschrieben bestimmt. Die durch LPS-Stimulation erhöhte Adhäsion ist evident (⌀ im Vergieich zu LPS, Zeitpunkt ⌀). Nach 24 Stunden war die erhöhte Adhäsion weitgehend durch N-Acetylhydroxyprolin gehemmt.

### Beispiel 3:

Die kurzfristige und langfristige Applikation von N-Acetylhydroxyprolin in Standardmodellen der Entzündungsforschung, wie dem Carrageenin-Pfotenödem, der Adjuvansarthritis und dem Baumwollgranulom, zeigten bei histologischer Aufarbeitung insbesondere eine deutliche reduzierte Invasion der Granulozyten (vgl. Tab. 2). Hingegen war die Invasion von monozytären Leukozyten kaum verändert.

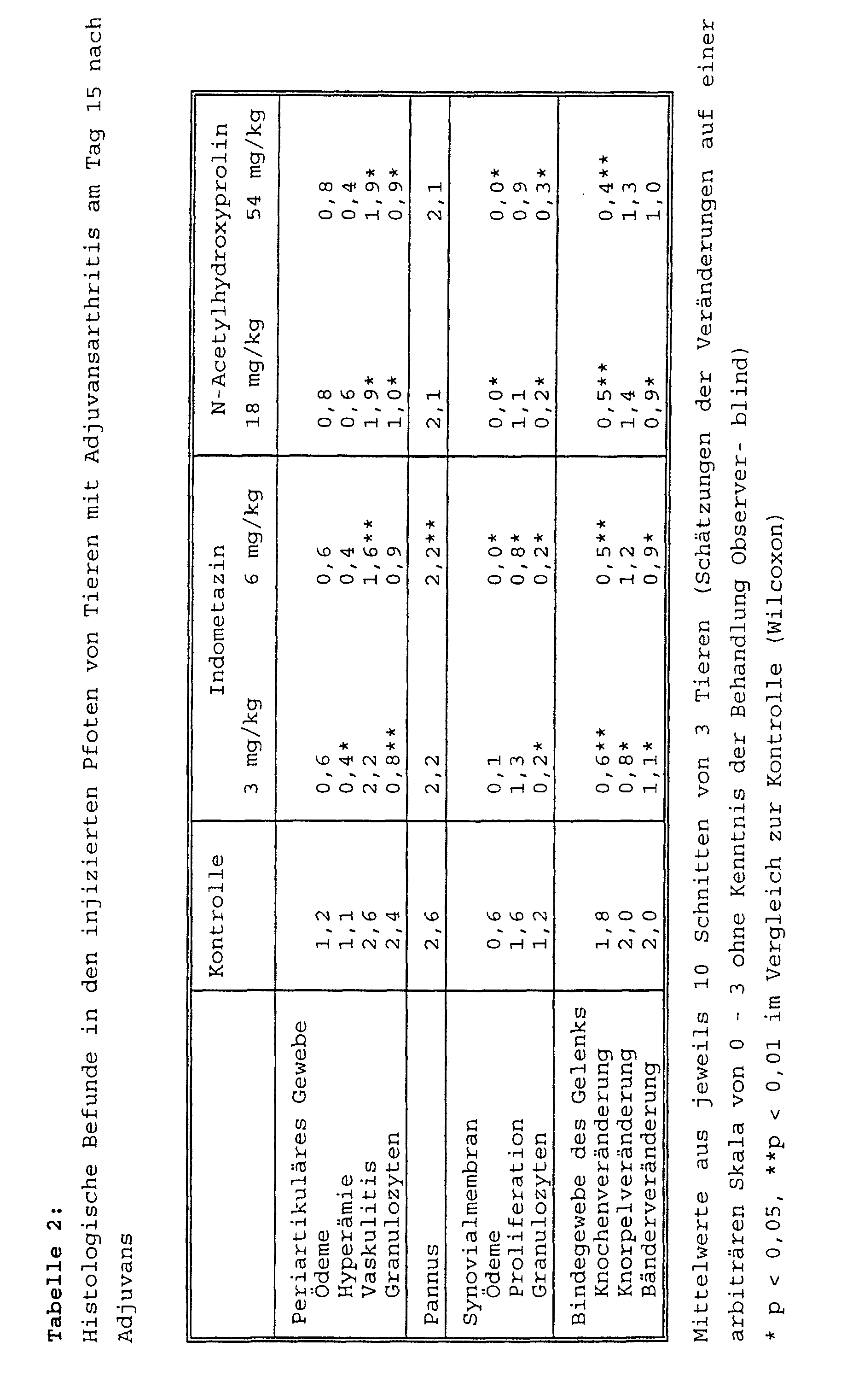

### Beispiel 4:

In Modellen der akuten allergischen Entzündung, wie z. B. dem durch Eiweiß im dafür sensibilisierten Meerschweinchen ausgelösten eosinophilen Lungeninfiltrat, zeigte N-Acetylhydroxyprolin keinen meßbaren Effekt auf die Einwanderung der eosinophilen Granulozyten. Die in der späten Phase beobachtbare quantifizierbare Einwanderung von Granulozyten hingegen war signifikant reduziert.

### Beispiel 5:

### Untersuchung der Wirkung von N-Acetylhydroxyprolin nach Ischämie im Tierversuch

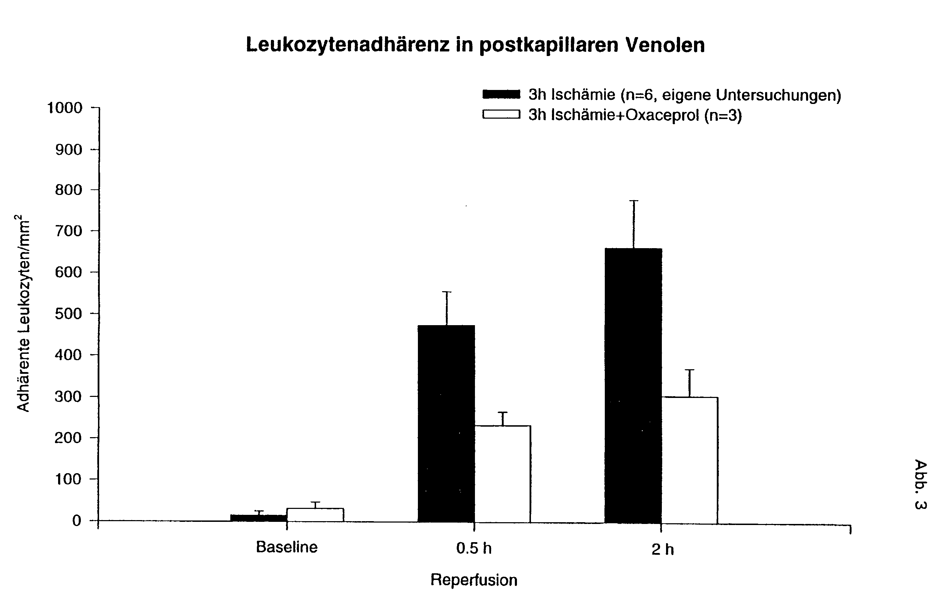

In dem von Nolte et al., Int. J. Microcirc. 15 (suppl 1) (1995), 9 - 16 beschriebenen Postischämiemodell beim Hamster zeigte sich, dass N-Acetylhydroxyprolin (Oxazeprol) bei Dosierungen von 50 mg/kg kurz yor Reperfusion (10 min) gegeben, die Adhäsion der Leukozyten am ischämisch traumatisierten Gefäßendothel um ca. 50 % reduzierte. Dieser Effekt war nach 0,5 und 2 Stunden vorhanden (Abb. 3). Er trat in allen untersuchten Tieren auf. Oxazeprol verhielt sich damit ähnlich wie Buflomidil (ein PDE4-Hemmer), Iloprostol (ein PhI-Analog) und monoklonale Antikörper gegen Selektine, d.h. N-Acetylhydroxyprolin verhinderte spezifisch die im Gefolge einer Noxe (Ischämie) auftretende Leukozytenadhäsion und -invasion ins Gewebe.

Diese Ergebnisse zeigen, dass Oxazeprol in vivo die Granulozytenadhäsion und damit wahrscheinlich auch die Emigration reduziert. Es lässt sich der Schluss ziehen, dass Oxazeprol Indikationen bei allen Erkrankungen hat, die durch aktivierte, evadierende Granulozyten aufrecht erhalten oder imitiert werden, z.B. entzündliche Gelenk- und Muskelerkrankungen, post-operative, posttraumatische und postinfektiöse Entzündungen, Postischämiesyndrome etc.

## Patentansprüche

1. Verwendung von Hydroxyprolinderivaten der Formel (I) 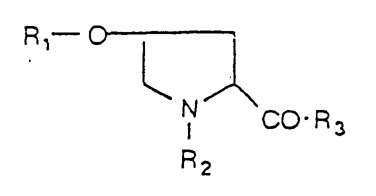 wobei R₁ und R₂ gleich oder verschieden sein können und Wasserstoff, Alkylgruppen mit 1 bis 10 C-Atomen oder eine Acylgruppe mit 1 bis 8 C-Atomen, R₃ eine Hydroxy-, Alkoxy- oder Aminogruppe, welche 1 oder 2 Alkylsubstituenten mit 1 bis 4 C-Atomen als auch 1 oder 2 Arylsubstituenten mit 6 bis 10 C-Atomen haben kann, bedeuten, oder eines pharmazeutisch verträglichen Salzes davon zur Herstellung eines Arzneimittels zur Prophylaxe und Heilung von Gewebeschäden, welche durch Invasion und Aktivierung von Leukozyten verursacht sind, ausgenommen rheumatische Entzündungen.

2. Verwendung nach Anspruch 1
**dadurch gekennzeichnet,**
**dass** das Arzneimittel zur Prophylaxe oder Behandlung von
a) akuten Abstoßungsreaktionen von Transplantaten,
b) Gewebeschäden im Gefolge von Herzinfarkt, Thrombosen, Embolien, oder
c) zur Verhinderung und Linderung von akuten Schüben exerzerbierender Hautkrankheiten wie Neurodermitis, Psoriasis,
d) posttraumatischen Gewebeschäden,
e) postoperativen Gewebeschäden und
f) akuten Schüben chronischer intestinaler Entzündungen und
g) septischen Erscheinungsformen
eingesetzt wird.

3. Verwendung gemäß Anspruch 1 oder 2,
**dadurch gekennzeichnet,**
**dass** in Formel (I) R₁ und R₂ H, Alkylgruppen oder Acylgruppen mit 1 bis 3 C-Atomen und R₃ OH bedeuten.

4. Verwendung nach Anspruch 1, 2 oder 3,
**dadurch gekennzeichnet,**
**dass** man N-Acetylhydroxyprolin verwendet.

## Claims

1. Use of hydroxyproline derivatives of formula (I) 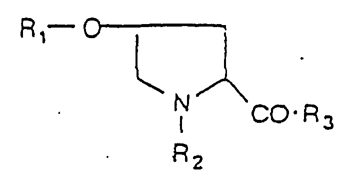 in which R₁ and R₂ can be the same or different and denote hydrogen, alkyl groups with 1 to 10 C atoms or an acyl group with 1 to 8 C atoms and R₃ denotes a hydroxy, alkoxy or amino group which can have 1 or 2 alkyl substituents with 1 to 4 C atoms as well as 1 or 2 aryl substituents with 6 to 10 C atoms or a pharmaceutically acceptable salt thereof to produce pharmaceutical preparation for the prophylaxis and healing of tissue damage which is caused by invasion and activation of leucocytes with the exception of rheumatic inflammations.

2. Use as claimed in claim 1,
**wherein**
the pharmaceutical preparation is used for the prophylaxis or treatment of
a) acute rejection reactions of transplants,
b) tissue damage as a result of cardiac infarction, thromboses, embolies,
c) to prevent or alleviate acute episodes of exacerbating skin diseases such as neurodermitis, psoriasis,
d) post-traumatic tissue damage,
e) post-operative tissue damage,
f) acute episodes of chronic intestinal inflammations and
g) septic manifestations.

3. Use as claimed in claim 1 or 2,
**wherein**
in formula I R₁ and R₂ denote H, alkyl groups or acyl groups with 1 to 3 C atoms and R₃ denotes OH.

4. Use as claimed in claim 1, 2 or 3,
**wherein**
N-acetylhydroxyproline is used.

## Revendications

1. Utilisation de dérivés d'hydroxyproline de formule (I) 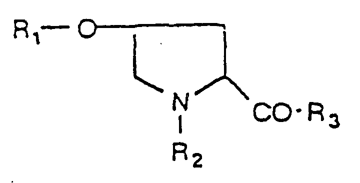 dans laquelle R₁ et R₂ peuvent être identiques ou différents et représentent l'hydrogène, des groupes alkyle de 1 à 10 atomes de carbone ou un groupe acyle de 1 à 8 atomes de carbone, R₃ représente un groupe hydroxy, alcoxy ou amino pouvant avoir 1 ou 2 substituants alkyle de 1 à 4 atomes de carbone ou 1 ou 2 substituants aryle de 6 à 10 atomes de carbone, ou d'un de leurs sels pharmaceutiquement acceptables, pour la préparation d'un médicament destiné à la prophylaxie ou au traitement de lésions tissulaires dues à une invasion et une activation de leucocytes, à l'exception d'inflammations rhumatismales.

2. Utilisation selon la revendication 1, **caractérisée en ce que** l'on utilise le médicament pour la prophylaxie ou le traitement
a) de réactions aiguës de rejet de greffes,
b) de lésions tissulaires à la suite d'un infarctus du myocarde, de thromboses, d'embolies ou
c) pour empêcher et soulager des crises aiguës de maladies de la peau évoluant par poussées comme la névrodermite, le psoriasis,
d) de lésions tissulaires post-traumatiques,
e) de lésions tissulaires post-opératoires,
f) de crises aiguës d'inflammations intestinales chroniques et
g) de manifestations septiques.

3. Utilisation selon la revendication 1 ou 2, **caractérisée en ce que**, dans la formule (I), R₁ et R₂ représentent H, des groupes alkyle ou des groupes acyle de 1 à 3 atomes de carbone et R₃ représente OH.

4. Utilisation selon la revendication 1, 2 ou 3, **caractérisée en ce que** l'on utilise la N-acétylhydroxyproline.
